(19) Europäisches Patentamt
European Patent Office
Office européen des brevets

(11) **EP 3 395 388 A1**

(12) **EUROPEAN PATENT APPLICATION**
published in accordance with Art. 153(4) EPC

(43) Date of publication:
**31.10.2018 Bulletin 2018/44**

(51) Int Cl.:
**A61M 16/01** *(2006.01)*   **A61M 16/00** *(2006.01)*

(21) Application number: **16877160.8**

(86) International application number:
**PCT/CN2016/079346**

(22) Date of filing: **15.04.2016**

(87) International publication number:
**WO 2017/107346 (29.06.2017 Gazette 2017/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **22.12.2015 CN 201510971328**

(71) Applicant: **Beijing Aeonmed Co., Ltd.
Beijing 100070 (CN)**

(72) Inventors:
• **HUA, Wei
  Beijing 100070 (CN)**
• **HAN, Wenlan
  Beijing 100070 (CN)**

(74) Representative: **Hanna Moore + Curley
Garryard House
25/26 Earlsfort Terrace
Dublin 2, D02 PX51 (IE)**

(54) **METHOD FOR CONTROLLING SLOPE RISE TIME IN PCV VENTILATION MODE OF TURBINE ANAESTHESIA APPARATUS**

(57)    Disclosed is a method for controlling the slope rise time in a PCV ventilation mode of a turbine anaesthesia apparatus, comprising: setting one quarter of the set slope rise time (Tslope) as an e-exponential function time constant; determining a control pressure value (Praise) according to the e-exponential function time constant; and increasing the turbine pressure according to the set control pressure value (Praise). Under the control of the control method, the turbine pressure rises quickly during the initial stage, but is smooth when approaching and reaching a target pressure (PTarget). Such a control method facilitates sufficient gas exchange between the patient's pulmonary alveoli and fresh air, so as to achieve a good effect.

Fig. 1

**Description**

**TECHNICAL FIELD**

The present invention relates to the field of anaesthesia apparatus control, and more specifically, to a method for controlling the slope rise time in a PCV ventilation mode of a turbine anaesthesia apparatus.

**BACKGROUND OF THE INVENTION**

The turbine anaesthesia apparatus is a type of anaesthesia apparatus that uses a turbine as a drive air source. In the pressure control ventilation PCV mode, the initial pressure rise stage (i.e. Tslope stage) is an important performance indicator in the PCV ventilation mode, which reflects that a target pressure can be quickly reached in a set time during the ventilation procedure. The speed of reaching the target pressure value can be controlled by adjusting the Tslope time.

In the current turbine PCV control methods, in order to control the Tslope pressure rise procedure, the main way adopted is to segment the target pressure within a specified rise time, and gradually accumulate the control pressure in each segment of the time so as to finally reach the set target pressure. A problem of such an approach is that the control pressure rises linearly, and when the Tslope time is set to be longer, it takes a longer time to reach the target value, which makes it impossible for a patient to get a target ventilation effect quickly in the clinical application of the anaesthesia apparatus, thus reducing the clinical effect of the PCV ventilation mode. In view of this, there is a need for technical improvement.

**SUMMARY OF THE INVENTION**

In order to overcome such drawbacks of the existing turbine PCV control methods in that the slope rise time is long, and thus a patient cannot get the target ventilation effect quickly and the clinical effect of the PCV ventilation mode is reduced, an objective of the present invention is to provide a method for controlling the slope rise time, which can raise the turbine pressure quickly.

To achieve the foregoing objective, the present invention provides a method for controlling the slope rise time in a PCV ventilation mode of a turbine anaesthesia apparatus, comprising:

step 1): setting an e-exponential function time constant, wherein
one quarter of the set slope rise time is used as thee-exponential function time constant;
step 2): determining a control pressure value according to the e-exponential function time constant set in step 1), wherein the control pressure value is calculated according to the equations below:

$$TCon = 1 / (Tslope / 4);$$

$$Praise = PTarget * (1 - \exp (0 - PassTime * TCon));$$

wherein, Tslope is the set slope rise time, Tslope / 4 is the e-exponential function time constant, TCon is an intermediate variable, PTarget is a set target pressure, Praise is the control pressure value outputted from the calculation, and PassTime represents the time that has passed from the initial turbine pressure rise stage;
step 3): increasing the turbine pressure according to the control pressure value set in step 2).

The present invention has the following advantages: under the control of the control method of the present invention, the turbine pressure rises quickly during the initial stage, but is smooth when approaching and reaching a target pressure. Such a control method facilitates sufficient gas exchange between the patient's pulmonary alveoli and fresh air, so as to achieve a good effect.

**BRIEF DESCRIPTION OF THE DRAWINGS**

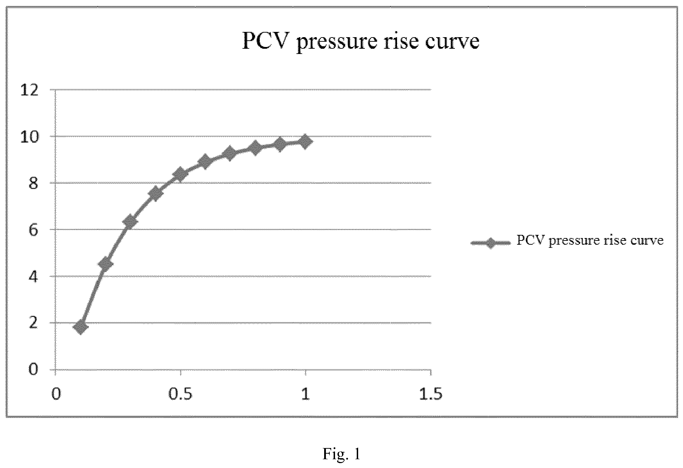

Fig. 1 is a waveform graph of a control pressure during a rise stage in a turbine PCV ventilation mode.

**DETAILED DESCRIPTION OF THE INVENTION**

Now the present invention is further described in conjunction with the accompanying drawing.

The basic principle of a method for controlling a turbine pressure at a rise stage according to the present invention lies in: calculating a control pressure value by an e-exponential function, and increasing the turbine pressure according to the control pressure value.

Due to properties of the e-exponential function, the turbine pressure will quickly rise to 80% of a target pressure and then reach the target pressure value smoothly. The suction pressure waveform shows that the suction pressure wave has a steep rise stage and a long plateau time without any peak.

The method of the present invention comprises:

step 1): setting an e-exponential function time constant;

in the present invention, one quarter of the slope rise time Tslope is used as the e-exponential function time constant;

step 2): determining a control pressure value according to the e-exponential function time constant set in step 1), wherein the control pressure value is calculated according to the equations below:

$$TCon = 1 / (Tslope / 4);$$

$$Praise = PTarget * (1 - \exp(0 - PassTime * TCon));$$

wherein, Tslope is the set slope rise time, (Tslope / 4) is the e-exponential function time constant, TCon is an intermediate variable, PTarget is a set target pressure, Praise is the control pressure value outputted from the calculation, and PassTime represents the time that has passed from the initial turbine pressure rise stage; when PassTime reaches the set slope rise time Tslope, i.e. 4 times of the time constant, the pressure value Praise will reach 98% of the target pressure value Ptarget;

step 3): increasing the turbine pressure according to the control pressure value set in step 2).

Table 1 below shows calculated control pressure data during the rise stage in the turbine PCV ventilation mode, wherein PTarget is set to be 10 cm $H_2O$, and Tslope is set to be 1 second. As seen from the table, when Tslope reaches the maximum time, i.e. 1 second, the calculated pressure value is 9.77 cm$H_2O$, i.e. 98% of the target pressure 10 cm$H_2O$.

Table 1

| No. | Tslope Rise time (second) | Pressure (cmH$_2$O) |
|---|---|---|
| 1 | 0.1 | 1.81 |
| 2 | 0.2 | 4.51 |
| 3 | 0.3 | 6.32 |
| 4 | 0.4 | 7.53 |
| 5 | 0.5 | 8.34 |
| 6 | 0.6 | 8.89 |
| 7 | 0.7 | 9.25 |
| 8 | 0.8 | 9.50 |
| 9 | 0.9 | 9.66 |
| 10 | 1 | 9.77 |

Fig. 1 is a wave form graph of a control pressure during a rise stage in a turbine PCV ventilation mode. As seen from the waveform graph of the control pressure, the control pressure rises quickly during the initial stage, but is smooth when approaching and reaching the target pressure. Such a control method facilitates sufficient gas exchange between the patient's pulmonary alveoli and fresh air, so as to achieve a good effect.

Finally, it should be explained that the foregoing embodiments are intended to merely illustrate rather than limit the technical solutions of the invention. While the present invention has been described in detail with reference to the embodiments, it shall be understood to those skilled in the art that various modifications or equivalent substitutions to the technical solutions of the present invention are within the scope of the claims of the present invention, without departing from the spirit and scope of the technical solutions of the invention.

**Claims**

1.  A method for controlling the slope rise time in a PCV ventilation mode of a turbine anaesthesia apparatus, comprising:

    step 1): setting an e-exponential function time constant, wherein
    one quarter of the set slope rise time is used as the e-exponential function time constant;
    step 2): determining a control pressure value according to the e-exponential function time constant set in step 1), wherein the control pressure value is calculated according to the equations below:

    $$TCon = 1 / (Tslope / 4);$$

    $$Praise = PTarget * (1 - exp (0 - PassTime * TCon));$$

    wherein, Tslope is the set slope rise time, Tslope / 4 is the e-exponential function time constant, TCon is an intermediate variable, PTarget is a set target pressure, Praise is the control pressure value outputted from the calculation, and PassTime represents the time that has passed from the initial turbine pressure rise stage;
    step 3): increasing the turbine pressure according to the control pressure value set in step 2).

Fig. 1

<div align="center">

# INTERNATIONAL SEARCH REPORT

</div>

| | International application No. |
|---|---|
| | **PCT/CN2016/079346** |

**A. CLASSIFICATION OF SUBJECT MATTER**

<div align="center">A61M 16/01 (2006.01) i</div>

According to International Patent Classification (IPC) or to both national classification and IPC

**B. FIELDS SEARCHED**

Minimum documentation searched (classification system followed by classification symbols)

<div align="center">A61M</div>

Documentation searched other than minimum documentation to the extent that such documents are included in the fields searched

Electronic data base consulted during the international search (name of data base and, where practicable, search terms used)

CNPAT, CNKI, WPI, EPODOC: BEIJING AEONMED CO., LTD.; HUAWEI; HAN, Wenlan; anesthesia, natural logarithm, PCV, e, EXP, pressure, ris+, rais+, time, breath+, natural, logarithm

**C. DOCUMENTS CONSIDERED TO BE RELEVANT**

| Category* | Citation of document, with indication, where appropriate, of the relevant passages | Relevant to claim No. |
|---|---|---|
| X | CN 102245243 A (ROYAL DUTCH PHILIPS ELECTRONICS LTD.), 16 November 2011 (16.11.2011), description, paragraphs [0019]-[0024] and [0043], and figures 1-3 | 1 |
| A | CN 102245246 A (ROYAL DUTCH PHILIPS ELECTRONICS LTD.), 16 November 2011 (16.11.2011), the whole document | 1 |
| A | CN 102266614 A (BEIJING AEONMED CO., LTD.), 07 December 2011 (07.12.2011), the whole document | 1 |
| A | EP 2165726 A2 (BORM, H.J.), 24 March 2010 (24.03.2010), the whole document | 1 |

☐ Further documents are listed in the continuation of Box C.     ☒ See patent family annex.

| | |
|---|---|
| \*    Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 23 May 2016 (23.05.2016) | **28 June 2016 (28.06.2016)** |

| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **WEN, Bo** Telephone No.: (86-10) **61648419** |
|---|---|

Form PCT/ISA/210 (second sheet) (July 2009)

## INTERNATIONAL SEARCH REPORT
### Information on patent family members

International application No.

**PCT/CN2016/079346**

| Patent Documents referred in the Report | Publication Date | Patent Family | Publication Date |
|---|---|---|---|
| CN 102245243 A | 16 November 2011 | CN 102245243 B | 25 February 2015 |
| | | AU 2009325991 B2 | 13 November 2014 |
| | | US 2011226248 A1 | 22 September 2011 |
| | | WO 2010067236 A1 | 17 June 2010 |
| | | AU 2009325991 A1 | 28 July 2011 |
| | | JP 5702727 B2 | 15 April 2015 |
| | | EP 2376160 A1 | 19 October 2011 |
| | | JP 2012511338 A | 24 May 2012 |
| | | IN CHENP201104709 E | 16 November 2012 |
| CN 102245246 A | 16 November 2011 | BR PI0917726 A2 | 16 February 2016 |
| | | WO 2010070498 A1 | 24 June 2010 |
| | | EP 2384211 B1 | 16 March 2016 |
| | | US 2011232643 A1 | 29 September 2011 |
| | | JP 2012512687 A | 07 June 2012 |
| | | EP 2384211 A1 | 09 November 2011 |
| | | JP 5749655 B2 | 15 July 2015 |
| | | IN 4957CHENP2011 A | 16 November 2012 |
| CN 102266614 A | 07 December 2011 | EA 201270805 A1 | 30 April 2013 |
| | | WO 2012089090 A1 | 05 July 2012 |
| | | EA 023035 B1 | 29 April 2016 |
| EP 2165726 A2 | 24 March 2010 | DE 102008048824 A1 | 25 March 2010 |

Form PCT/ISA/210 (patent family annex) (July 2009)